# EUROPEAN PATENT SPECIFICATION

(11) **EP 0 354 973 B1**
(45) Date of publication and mention of the grant of the patent: **15.03.1995**
(21) Application number: 88307563.2
(22) Date of filing: 15.08.1988
(51) Int. Cl.: A61K 9/16, A61K 9/50

(54) **Drug delivery system**
Arzneimittelverabreichungssystem
Système de délivrance de médicaments

(43) Date of publication of application: 21.02.1990
(73) Proprietor: K.V. PHARMACEUTICALS, INC., St. Louis Missouri 63144 (US)
(72) Inventor: Paradissis, George N. c/o K.V. Pharmaceuticals Inc, St. Louis Missouri 63144 (US)
(74) Representative: Mütschele, Thomas

(56) References cited:
- EP-A- 0 247 634
- BE-A- 555 932

## Description

### Background of the Invention

### I. Field of the Invention

The invention relates to the delivery of drugs which do not have a palatable taste and where it is undesirable to ingest said drugs in a solid state. Those of ordinary skill in the art would have expertise in taste masking drugs and related products.

### II. Description of the Prior Art

Taste masking of drugs through the use of flavors is well known. However, flavoring ingredients are not sufficient to mask certain drugs and vitamins. Therefore, physical means have been developed to encapsulate or place in a chemical barrier these drugs to mask their undesirable taste characteristics.

One such system which has been used for vitamins is disclosed in U.S. Patents 3,037,911; 3,080,292; 3,080,293; and 3,279,994. These methods produce products which are in beadlet form. Although these beadlets are effective for taste masking they can cause other difficulties with respect to certain drugs, and types of patients. For instance, patients who have problems taking solid dosage forms cannot utilize drugs produced by this beadlet process.

Also, products, such as potassium chloride cannot effectively be taken in the beadlet form described by the prior art. Solid forms of potassium chloride are known to lay along the stomach or intestinal lining and ulcerate the stomach or intestinal wall. This ulceration or bleeding of the stomach is a highly undesirable side effect, especially for elderly patients. Thus, the treatment of one affliction causes the occurrence of other serious medical problems.

BE-A-555932 describes a pharmaceutical composition comprising granules which contain the active agent and granules which contain a sweetening agent, wherein the different granules are connected by an eatable rubber. The granules containing the sweetening agent break immediately after contact with a liquid whereas the granules containing the active agent remain intact for a longer time. Furthermore, an aroma-providing agent is in the interstices between the different granules. The sweetening agent which is released into the liquid and the aroma-providing agent serves for masking the bad taste of the portion of active agent which already has been released into the liquid.

EP-A-0 247 634 discloses a pharmaceutical in the form of small pellets having a taste- and odor-masking coating of a polymer practically insoluble in the mouth but readily soluble in an acid environment such as in the stomach. Said coating is comprised of a copolymer containing residues of (meth)acrylic esters and dimethyl aminoethyl (meth)acrylate, and an acid to solubilize the polymeric component (claim 1) which acid is selected from hydrochloric, nitric or sulfuric acid or from organic acids. For administration a premeasured dosage of the pellets is admixed with a food or other consumable material that is convenient and/or palatable to the patient.

The invention relates to a pharmaceutical composition as defined in the claims.

The present invention is advantageous since it allows the delivery of taste masked particles in a liquid form. It is also advantageous since it is economical and provides a product which has commercially desirable properties. Further, it is advantageous since it does not affect the active properties of the drug which is being delivered. Other advantages include being able to provide a solid drug form to those who cannot or will not take such drugs especially in large quantities. Finally, it should be noted that the composition of the invention meets a long felt need in the art.

### Description of the Preferred Embodiments

The basic particles are produced by the methods disclosed by U.S. Patents 3, 037, 911; 3, 080, 292; 3, 080, 293; and 3, 279, 994.

The beads produced by these processes are hydrophobic and therefore are unsuitable for use in formulating liquid suspensions.

The beads are formed by pulverizing the active agents. The preferred active agent is potassium chloride which is pulverized to approximately 0,074 mm (200 mesh). Waxes or preferably a mixture of mono and diglycerides are melted to a molten state. For glycerides this is approximately 60-80 degrees C. The active agent is added to the melted mixture to form 40-60% of the total.

The waxes and glycerides provide a taste masking coating over the active ingredient. In the present invention the densities of taste masking coating and the active agents are balanced. The total density of the product approximates that of a carrier liquid in which it is to be suspended and should preferably equal the density of the liquid into which it is to be suspended so as to form a drinkable suspension. Thus, if the product is to be suspended in water its overall density should approximate 1. Also, the ratio taste masking coating to active agent should be adjusted to reduce the solubility of the drug in the first 60 seconds.

Flavors, modifiers and/or air are added to the mixture. The mixture is placed in a spinner which ejects liquid beads of the mixture which congeals as it cools. The beads are usually 0,149 - 0,59 mm (30 - 100 mesh). They can be compressed into tablet form.

The taste masking coating is hydrophobic and so the beadlets will float on water. In order to render them hydrophilic and improve workability they are blended with surfactants or surface modifiers. A preferred surfactant is sodium lauryl sulfate which forms 0.25 to 2%, preferably 1%, of the beadlet by weight. The surfactant could be added either before or after the spinning of the beads.

Also, additional colors and flavors can be added. Anticaking agents, such as, silicon dioxide are advantageously added and may comprise 1-3% by weight of the bead. Other materials which do not affect the basic properties of the material may be incorporated into the beadlets. It may be especially desirable to add pH modifiers which reduce dissolution initially but are neutralized by gastric pH, thereby, allowing rapid dissolution in the stomach.

The beadlets may be packaged in single dose packages. When the patient is required to take a dose, the contents of the package are emptied into a glass of liquid. The beadlets form a suspension and do not dissolve immediately. However, they do dissolve fairly rapidly, i.e. no less than 90% within 15 minutes, in order to avoid gastric ulceration. The patient drinks the liquid.

This system has a number of advantages. First, the active ingredient is taste masked as in the solid dosage forms. Second, the liquid can be taken by those who are not capable of taking a solid dosage form. Third, the side effects of the solid dosage form are not encountered. Fourth, gastric ulceration does not occur due to the rapid dissolution of the product. And fifth, a more acceptable consumer delivery system is achieved which encourages patient compliance in taking drugs, especially when large dosages are required.

### (EXAMPLE ONE)

### ALBUTEROL SULFATE CONTROLLED RELEASE LIQUETTE™

The Albuterol Sulfate Controlled Release Liquette™ is composed of:

| | |
|---|---|
| Albuterol Sulfate | 7.5% |
| Hydrogenated Vegetable Oil | 92.5% |

The fatty base is melted and the albuterol sulfate powder is added carefully.

The resulted melt is congealed by means of a spinning disc or other suitable spray congealing equipment.

The resulting beads are blended with silicon dioxide and sodium lauryl sulfate to yield the liquette™.

| | |
|---|---|
| Albuterol Sulfate Beads | 99.25% |
| Silicon Dioxide | 0.25% |
| Sodium Lauryl Sulfate | 0.50% |

### (EXAMPLE TWO)

### THEOPHYLLINE CONTROLLED RELEASE LIQUETTE™

The Theophylline Controlled Release Liquette™ is composed of:

| | |
|---|---|
| Theophylline | 40% |
| Carnauba Wax | 60% |

The wax base is melted and the theophylline powder is added carefully.

The resulted melt is congealed by means of a spinning disc or other suitable spray congealing equipment.

The resulting beads are blended with silicon dioxide and sodium lauryl sulfate to yield the liquette™.

| | |
|---|---|
| Theophylline Beads | 99.25% |
| Silicon Dioxide | 0.25% |
| Sodium Lauryl Sulfate | 0.50% |

### (EXAMPLE THREE)

### ANTACID LIQUETTE™

The antacid Liquette™ is composed of three separate type of beads which have the following composition:

| Bead #1 | % w/w |
|---|---|
| Magnesium Hydroxide | 21.31 |
| Aluminum Hydroxide Dried Gel | 24.03 |
| Na Azulene Sulfonate | 0.19 |
| Na Cu Chlorophyllin | 0.21 |
| Mono and Diglycerides | 26.40 |
| Carnauba Wax | 26.40 |
| Na Lauryl Sulfate | 1.46 |
| | 100.00 |

| Bead #2 | |
|---|---|
| Scopolis 10% Extract | 60.00 |
| Mono and Diglycerides | 39.90 |
| FD&C Blue #1 | 0.06 |
| FD&C Yellow #5 | 0.04 |
| | 100.00 |

| Bead #3 | |
|---|---|
| Na Cu Chlorophyllin | 33.00 |
| Stearic Acid | 67.00 |
| | 100.00 |

The base (carnauba wax, mono and diglycerides and stearic acid) is melted and the active ingredients are added carefully.

The resulted melt is congealed by means of a spinning disc or other suitable spray congealinig equipment.

The resulted beads are blended in the following ratio:

| | |
|---|---|
| Bead #1: | 13.5 parts |
| Bead #2: | 2.0 parts |
| Bead #3: | 1.0 parts |

To the aforementioned blend, the following materials are added and the entire mixture is re-blended.

| | |
|---|---|
| Silicon Dioxide | 0.50% |
| Menthol Flavor | 0.40% |
| Peppermint Flavor | 0.07% |
| Sodium Lauryl Sulfate | 0.50% |

## Claims (Claims for the following Contracting State(s): AT, BE, CH, LI, DE, FR, GB, GR, IT, LU, NL, SE)

1. A taste-masked pharmaceutical composition in the form of a suspension of hydrophilic particles in a liquid carrier, said particles comprising a pharmaceutically active agent **characterized in that** said particles are beads, wherein said beads comprise said active agent, a surfactant or a surface modifier and a coating selected from waxes and mixtures of monoglycerides and diglycerides, the density of said beads approximating that of said liquid carrier so as to form a drinkable suspension.

2. The pharmaceutical composition of claim 1, characterized in that said active agent comprises potassium chloride.

3. The pharmaceutical composition of claim 1 or 2, characterized in that said beads further comprise from 1% to 3% by weight of an anti-caking agent.

4. The pharmaceutical composition of claim 3, characterized in that said anti-caking agent is silicon dioxide.

5. The pharmaceutical composition of any of claims 1-4, characterized in that said beads further comprise one or more materials selected from colors, pH modifiers and flavors.

6. The pharmaceutical composition of any of claims 1-5, characterized in that said surfactant is sodium lauryl sulfate and is present from 0.25% to 2% by weight, based on the total weight of the beads.

7. The pharmaceutical composition of any of claims 1-6, characterized in that said beads dissolve by not less than 90% within 15 minutes following liquid carrier contact.

8. The pharmaceutical composition of any of claims 1-7, characterized in that said liquid carrier is water and said surfactant is sodium lauryl sulphate

9. Hydrophilic particles comprising a pharmaceutically active agent for the preparation of a suspension of said hydrophilic particles in a liquid carrier, characterized in that said particles are beads wherein said beads comprise said active agent, a surfactant or a surface modifier and a coating selected from waxes and mixtures of monoglycerides and diglycerides, the density of said beads approximating that of said liquid carriers so as to form a drinkable suspension.

10. The hydrophilic particles of claim 9 being defined as in any of claims 2 - 7.

11. The hydrophilic particles of claim 9 or 10, wherein said surfactant is sodium lauryl sulphate.

## Claims (Claims for the following Contracting State(s): ES)

1. A method for preparing a taste-masked pharmaceutical composition by forming a suspension of hydrophilic particles in a liquid carrier, said particles comprising a pharmaceutically active agent, wherein said particles are beads, wherein said beads comprise said active agent, a surfactant or a surface modifier and a coating selected from waxes and mixtures of monoglycerides and diglycerides, the density of said beads approximating that of said liquid carrier so as to form a drinkable suspension.

2. The method of claim 1 characterized in that said active agent comprises potassium chloride.

3. The method of claim 1 or 2, characterized in that said beads further comprise from 1% to 3% by weight of an anti-caking agent.

4. The method of claim 3, characterized in that as said anti-caking agent silicon dioxide is used.

5. The method of any of claims 1 to 4, characterized in that said beads further comprise one or more materials selected from colors, pH modifiers and flavors.

6. The method of any of claims 1 to 5, characterized in that as said surfactant sodium lauryl sulphate is used and is present from 0.25% to 2% by weight, based on the total weight of the beads.

7. The method of any of claims 1 to 6, characterized in that said beads dissolve by not less than 90% within 15 minutes following liquid carrier contact.

8. The method of any of claims 1 to 7, characterized in that as said liquid carrier water is used and as said surfactant sodium lauryl sulphate is used.

9. A method for preparing hydrophilic particles by pulverizing a pharmaceutically active agent, adding said agent to molten waxes or a molten mixture of mono- and diglycerides to provide a taste masking coating over the agent and blending said coating with surfactants or surface modifiers, characterized in that said particles are beads wherein said beads comprise said active agent, a surfactant or a surface modifier and a coating selected from waxes and mixtures of monoglycerides and diglycerides, the density of said beads approximating that of said liquid carrier so as to form a drinkable suspension.

10. The method of claim 9 being defined as in any of claims 2 to 7.

11. The method of claim 9 or 10, wherein as said surfactant sodium lauryl sulphate is used.

## Patentansprüche (Patentansprüche für folgende(n) Vertragsstaat(en): AT, BE, CH, LI, DE, FR, GB, GR, IT, LU, NL, SE)

1. Geschmacksmaskierte pharmazeutische Zusammensetzung in Form einer Suspension von hydrophilen Partikeln in einem flüssigen Träger, wobei die Partikel einen pharmazeutischen Wirkstoff enthalten, dadurch gekennzeichnet, daß die Partikel Kügelchen sind, worin die Kügelchen den Wirkstoff, ein oberflächenaktives Mittel oder ein Oberflächenmodifizierungsmittel und eine Beschichtung ausgewählt aus Wachsen und Mischungen von Monoglyceriden und Diglyceriden enthalten, wobei die Dichte der Kügelchen ungefähr der des flüssigen Trägers entspricht, so daß sich eine trinkbare Suspension ausbildet.

2. Pharmazeutische Zusammensetzung nach Anspruch 1, dadurch gekennzeichnet, daß der Wirkstoff Kaliumchlorid enthält.

3. Pharmazeutische Zusammensetzung nach Anspruch 1 oder 2, dadurch gekennzeichnet, daß die Kügelchen ferner von 1 Gew.-% bis 3 Gew.-% eines Mittels gegen Verklumpen enthalten.

4. Pharmazeutische Zusammensetzung nach Anspruch 3, dadurch gekennzeichnet, daß das Mittel gegen Verklumpen Siliciumdioxid ist.

5. Pharmazeutische Zusammensetzung nach einem der Ansprüche 1 bis 4, dadurch gekennzeichnet, daß die Kügelchen ferner einen oder mehrere Stoffe ausgewählt aus Farben, pH-Modifizierungsmitteln und Geschmackstoffen enthalten.

6. Pharmazeutische Zusammensetzung nach einem der Ansprüche 1 bis 5, dadurch gekennzeichnet, daß das oberflächenaktive Mittel Natriumlaurylsulfat ist und in 0,25 Gew.-% bis 2 Gew.-%, bezogen auf das Gesamtgewicht der Kügelchen, vorhanden ist.

7. Pharmazeutische Zusammensetzung nach einem der Ansprüche 1 bis 6, dadurch gekennzeichnet, daß die Kügelchen sich zu nicht weniger als 90 % innerhalb von 15 Minuten nach Kontakt mit flüssigem Träger auflösen.

8. Pharmazeutische Zusammensetzung nach einem der Ansprüche 1 bis 7, dadurch gekennzeichnet, daß der flüssige Träger Wasser ist und das oberflächenaktive Mittel Natriumlaurylsulfat.

9. Hydrophile Partikel, die einen pharmazeutischen Wirkstoff zur Herstellung einer Suspension aus den hydrophilen Partikeln in einem flüssigen Träger enthalten, dadurch gekennzeichnet, daß die Partikel Kügelchen sind, worin die Kügelchen den Wirkstoff, ein oberflächenaktives Mittel oder ein Oberflächenmodifizierungsmittel und eine Beschichtung ausgewählt aus Wachsen und Mischungen von Monoglyceriden und Diglyceriden enthalten, wobei die Dichte der Kügelchen ungefähr der des flüssigen Trägers entspricht, so daß sich eine trinkbare Suspension ausbildet.

10. Hydrophile Partikel nach Anspruch 9, die nach einem der Ansprüche 2 bis 7 definiert sind.

11. Hydrophile Partikel nach Anspruch 9 oder 10, worin das oberflächenaktive Mittel Natriumlaurylsulfat ist.

## Patentansprüche (Patentansprüche für folgende(n) Vertragsstaat(en): ES)

1. Verfahren zur Herstellung einer geschmacksmaskierten pharmazeutischen Zusammensetzung durch Bilden einer Suspension von hydrophilen Partikeln in einem flüssigen Träger, wobei die Partikel einen pharmazeutischen Wirkstoff enthalten, worin die Partikel Kügelchen sind, worin die Kügelchen den Wirkstoff, ein oberflächenaktives Mittel oder ein Oberflächenmodifizierungsmittel und eine Beschichtung ausgewählt aus Wachsen und Mischungen von Monoglyceriden und Diglyceriden enthalten, wobei die Dichte der Kügelchen ungefähr der des flüssigen Trägers entspricht, um eine trinkbare Suspension zu bilden.

2. Verfahren nach Anspruch 1, dadurch gekennzeichnet, daß der Wirkstoff Kaliumchlorid enthält.

3. Verfahren nach Anspruch 1 oder 2, dadurch gekennzeichnet, daß die Kügelchen ferner von 1 Gew.-% bis 3 Gew.-% eines Mittels gegen Verklumpen enthalten.

4. Verfahren nach Anspruch 3, dadurch gekennzeichnet, daß das als Mittel gegen Verklumpen Siliciumdioxid verwendet wird.

5. Verfahren nach einem der Ansprüche 1 bis 4, dadurch gekennzeichnet, daß die Kügelchen ferner einen oder mehrere Stoffe ausgewählt aus Farben, pH-Modifizierungsmitteln und Geschmackstoffen enthalten.

6. Verfahren nach einem der Ansprüche 1 bis 5, dadurch gekennzeichnet, daß als oberflächenaktives Mittel Natriumlaurylsulfat verwendet wird und von 0,25 Gew.-% bis 2 Gew.-%, bezogen auf das Gesamtgewicht der Kügelchen, vorhanden ist.

7. Verfahren nach einem der Ansprüche 1 bis 6, dadurch gekennzeichnet, daß die Kügelchen sich zu nicht weniger als 90 % innerhalb von 15 Minuten nach Kontakt mit flüssigem Träger auflösen.

8. Verfahren nach einem der Ansprüche 1 bis 7, dadurch gekennzeichnet, daß als flüssiger Träger Wasser verwendet wird und als oberflächenaktives Mittel Natriumlaurylsulfat verwendet wird.

9. Verfahren zur Herstellung hydrophiler Partikel durch Pulverisieren eines pharmazeutischen Wirkstoffes und Zugeben des Mittels zu geschmolzenen Wachsen oder einer geschmolzenen Mischung von Mono- und Diglyceriden, um eine Geschmack maskierende Beschichtung über den Wirkstoff aufzubringen und Mischen der Beschichtung mit oberflächenaktiven Mitteln oder Oberflächenmodifizierungsmitteln, dadurch gekennzeichnet, daß die Partikel Kügelchen sind, worin die Kügelchen den Wirkstoff, ein oberflächenaktives Mittel oder ein Oberflächenmodifizierungsmittel und eine Beschichtung ausgewählt aus Wachsen und Mischungen von Monoglyceriden und Diglyceriden enthalten, wobei die Dichte der Kügelchen ungefähr der des flüssigen Trägers entspricht, um eine trinkbare Suspension zu bilden.

10. Verfahren nach Anspruch 9 definiert nach einem der Ansprüche 2 bis 7.

11. Verfahren nach Anspruch 9 oder 10, worin als oberflächenaktives Mittel Natriumlaurylsulfat verwendet wird.

## Revendications (Revendications pour l'(les) Etat(s) contractant(s) suivant(s): AT, BE, CH, LI, DE, FR, GB, GR, IT, LU, NL, SE)

1. Composition pharmaceutique à goût masqué se présentant sous la forme d'une suspension de particules hydrophiles dans un véhicule liquide, lesdites particules comprenant un agent pharmaceutiquement actif, caractérisée par le fait que lesdites particules sont des perles, lesdites perles comprenant ledit agent actif, un agent tensio-actif ou un agent modificateur de surface et un enrobage choisi parmi les cires et les mélanges de monoglycérides et diglycérides, la densité desdites perles s'approchant de celle dudit véhicule liquide de façon à former une suspension buvable.

2. Composition pharmaceutique selon la revendication 1, caractérisée par le fait que ledite agent actif comprend du chlorure de potassium.

3. Composition pharmaceutique selon la revendication 1 ou 2 caractérisée par le fait que lesdites perles comprennent en outre de 1 % à 3% en poids d'un agent anti-agglutination.

4. Composition pharmaceutique selon la revendication 3, caractérisée par le fait que ledit agent anti-agglutination est du dioxyde de silicium.

5. Composition pharmaceutique selon l'une des revendications 1 à 4, caractérisée par le fait que lesdites perles comprennent en outre une ou plusieurs matières choisies parmi les colorants, les agents modificateurs de pH et les arômes.

6. Composition pharmaceutique selon l'une des revendications 1 à 5, caractérisée par le fait que ledit agent tensio-actif est le lauryl sulfate de sodium et est présent à raison de 0,25 % à 2 % en poids, sur la base du poids total des perles.

7. Composition pharmaceutique salon l'une des revendications 1 à 6, caractérisée par le fait que lesdites perles se dissolvent de pas moins de 90 % en l'espace de 15 minutes après mise en contact avec le véhicule liquide.

8. Composition pharmaceutique selon l'une des revendication 1 à 7, caractérisée par le fait que ledit véhicule liquide est de l'eau, et ledit agent tensio-actif est le lauryl sulfate de sodium.

9. Particules hydrophiles comprenant un agent pharmaceutiquement actif pour la préparation d'une suspension desdites particules hydrophiles dans un véhicule liquide, caractérisées par le fait que lesdites particules sont des perles, lesdites perles comprenant ledit agent actif, un agent tensio-actif ou un agent modificateur de surface et un enrobage choisi parmi les cires et les mélanges de monoglycérides et diglycérides, la densité desdites perles s'approchant de celle dudit véhicule liquide, de façon à former une suspension buvable.

10. Particules hydrophiles selon la revendication 9, définies comme à l'une des revendications 2 à 7.

11. Particules hydrophiles selon la revendication 9 ou 10, dans lesquelles ledit agent tensio-actif est le lauryl sulfate de sodium.

## Revendications (Revendications pour l'(les) Etat(s) contractant(s) suivant(s): ES)

1. Procédé de préparation d'une composition pharmaceutique à goût masqué par formation d'une suspension de particules hydrophiles dans un véhicule liquide, lesdites particules comprenant un agent pharmaceutiquement actif, lesdites particules étant des perles, lesdites perles comprenant ledit agent actif, un agent tensio-actif ou un agent modificateur de surface et un enrobage choisi parmi les cires et les mélanges de monoglycérides et diglycérides, la densité desdites perles s'approchant de celle dudit véhicule liquide de façon à former une suspension buvable.

2. Procédé selon la revendication 1, caractérisé par le fait que ledit agent actif comprend du chlorure de potassium.

3. Procédé selon la revendication 1 ou 2, caractérisé par le fait que lesdites perles comprennent en outre de 1 % à 3 % en poids d'un agent anti-agglutination.

4. Procédé selon la revendication 3, caractérisé par le fait que du dioxyde de silicium est utilisé en tant que ledit agent anti-agglutination.

5. Procédé selon l'une des revendications 1 à 4, caractérisé par le fait que lesdites perles comprennent en outre une ou plusieurs matières choisies parmi les colorants, les agents modificateurs de pH et les arômes.

6. Procédé selon l'une des revendications 1 à 5, caractérisé par le fait qu'en tant que ledit agent tensio-actif, du lauryl sulfate de sodium est utilisé et est présent à raison de 0,25 % à 2 % en poids, sur la base du poids total des perles.

7. Procédé selon l'une des revendications 1 à 6, caractérisé par le fait que lesdites perles se dissolvent de pas moins de 90 % en l'espace de 15 minutes après mise en contact avec le véhicule liquide.

8. Procédé selon l'une des revendications 1 à 7, caractérisé par le fait que de l'eau est utilisée en tant que ledit véhicule liquide, et du lauryl sulfate de sodium est utilisé en tant que ledit agent tensio-actif.

9. Procédé de préparation de particules hydrophiles par pulvérisation d'un agent pharmaceutiquement actif, addition dudit agent à des cires fondues ou à un mélange fondu de mono- et diglycérides pour réaliser un enrobage masquant le goût sur l'agent, et mélange dudit enrobage avec des agents tensio-actifs ou des agents modificateurs de surface, caractérisé par le fait que lesdites particules sont des perles, lesdites perles comprenant ledit agent actif, un agent tensio-actif ou un agent modificateur de surface et un enrobage choisi parmi les cires et les mélanges de monoglycérides et diglycérides, la densité desdites perles s'approchant de celle dudit véhicule liquide, de façon à former une suspension buvable.

10. Procédé selon la revendication 9, défini comme dans l'une des revendications 2 à 7.

11. Procédé selon la revendication 9 ou 10, dans lequel le lauryl sulfate de sodium est utilisé en tant que ledit agent tensio-actif.
